# EUROPEAN PATENT SPECIFICATION

(11) **EP 1 589 883 B1**
(45) Date of publication and mention of the grant of the patent: **29.12.2010**
(21) Application number: 04704293.2
(22) Date of filing: 22.01.2004
(51) Int. Cl.: A61B 17/17

(54) **Device for the determination of the holes of intramedullary nail**
Vorrichtung zur Bestimmung der Löcher eines Marknagels
Dispositif pour determiner les trous d'un clou centromedullaire

(30) Priority: 03.02.2003 HU 0300277
(43) Date of publication of application: 02.11.2005
(73) Proprietor: Sanatmetal Kft., 3300 Eger (HU)
(72) Inventor: FARKAS, József, H-3300 Eger (HU); SMUCZER, Tibor, H-3300 Eger (HU)
(74) Representative: Ronaszéki, Tibor
(86) International application number: PCT/HU2004/000004
(87) International publication number: WO 2004/069063

(56) References cited:
- EP-A- 0 135 804
- EP-A- 0 628 287
- US-A- 5 127 913
- US-A- 5 433 720
- US-A- 5 695 513

## Description

The subject of the invention relates to an auxiliary device for the determination of the positions of the holes of intramedullary nails which contains a source unit suitable for emitting signals penetrating body tissues and a sensor unit co-operating with the source unit and an internal positioning part-unit that can be connected to the intramedullary nail, either the source unit or the sensor unit is attached to the internal positioning part-unit, and the sensor unit is provided with a relay unit for receiving the signals of the source unit and a display unit connected to the relay unit through an information transmission line.

In the course of surgical operation, when it comes to restoring fractures of bones mechanical aiming devices are widely used to make it easier to find the locking holes created in the intramedullary nails placed in the bone. However, such aiming devices can only be used successfully in the case of slightly deformed intramedullary nails. In the case of long tubular bones such as arm and leg bones, in certain cases the intramedullary nails inserted into the medullary canal exhibits significant deformation, which significantly reduces the chance of finding the locking holes precisely.

The essence of the presently used aiming devices is that the area of operation is exposed to roentgen or gamma radiation, and the operation is monitored and the position of the locking holes of the intramedullary nail inserted in the bone is determined with an image-intensifying device or X-ray picture projecting equipment. Publication document number HU T/76.155. also represents such an image-intensifying solution, while patent description registration number HU 166.829 relates to an intermittently operated X-ray apparatus providing a continuous image.

Publication document number HU T/73.698. describes an aiming device in the case of which the area of operation is scanned with collimated beam of an encased radioactive isotope, and a part of the beam starting from the signalling device which penetrates the tissues is captured and evaluated with a detector.

Document EP 0 135 804 A, from which the preamble of claim 1 emanates, discloses an auxiliary device for the determination of the positions of the holes of intramedullary nails. It includes a source unit for emitting signals penetrating body tissues, a sensor unit which co-operates with the source unit and an internal positioning part-unit which can be connected to the intramedullary nail.

Document US 5 433 720 A describes an external positioning part-unit which determines the position of the holes with the help of magnetic fields.

However, the basic disadvantage of the known solutions is that radiation, which dangerous to the living organism, is used for the determination of the position, so the time of using the device ought to be limited, which is very disadvantageous in the case of larger operations.

A further disadvantage is that apart from the patient the radiation also endangers the operating surgeon, so their time spent in the operating theatre is also limited, which may cause delays in medical attendance.

It is also regarded as a disadvantage that the presently used auxiliary devices have a complex structure, they are difficult to handle and expensive to operate.

With the solution according to the invention our aim was to overcome the deficiencies of the known auxiliary devices used for the determination of the positions of locking holes and to create a version which is easy to use, does not result in any radiation exposure endangering the patient and the operating surgeon.

The basis of the idea behind the invention was formed by the recognition that if the intramedullary nail itself is used as a base unit and one member of the receiver-transmitter unit of the position determining device is inserted in the internal space of the intramedullary nail, while the other member is moved at a given distance along a precisely measurable and determinable path beside the unit placed in the intramedullary nail so that a slightly modified form of the aiming device used for making holes is used for determining the path of motion, then on the basis of the signals of the receiver the position of the deformed intramedullary nail can be determined with the desired precision without having to check, measure or determine the position of the locking bores with other devices, on a monitor in the form of a visible image, and so the task can be solved.

In accordance with the set aim the auxiliary device for the determination of the positions of the holes of intramedullary nails, which contains a source unit suitable for emitting signals penetrating body tissues and a sensor unit co-operating with the source unit and an internal positioning part-unit that can be connected to the intramedullary nail, either the source unit or the sensor unit is attached to the internal positioning part-unit, and the sensor unit is provided with a relay unit for receiving the signals of the source unit and a display unit connected to the relay unit through an information transmission line, is constructed in such a way that it is provided with an external positioning part-unit that can be connected to the intramedullary nail, the external positioning part-unit has a supporting structure with a fixing end and a free end and a motion member that can be connected to the free end of the supporting structure, the fixing end of the supporting structure is attached to the intramedullary nail in a fixed position, while the other from the source unit and the sensor unit is attached to the motion member of the external positioning part-unit, so the relay unit of the sensor unit and the source unit are interlinked to each other that is suitable for the continuous control and the exact determination of the relative position between the sensor unit and the source unit by the aid of the external positioning part-unit that is connected to the intramedullary nail.

A further criterion of the auxiliary device according to the invention may be that the internal positioning part-unit is a guide sleeve situated in the vicinity of the entrance point of the intramedullary nail, equipped with an orientation piece, running along at least a part of the length of the intramedullary nail.

In a possible version of the auxiliary device the sensor unit is supplemented with a carrying body comprising the relay unit and the display unit, and the carrying body has a covering surface suiting the shape and size of the guide sleeve, which can be at least partly inserted in the guide sleeve.

In another different construction of the invention the covering surface of the carrying body of the sensor unit is equipped with a locking piece co-operating with the orientation piece of the guide sleeve of the internal positioning part-unit situated in the intramedullary nail.

In a further construction example of the auxiliary device the display unit of the sensor unit is provided with a light emitting member and/or a sound emitting member and an energy source.

From the aspect of the invention it may be favourable, if the source unit has a natural magnet creating a magnetic field or a radiation source suitable for emitting radiation with a wavelength penetrating tissue.

In a further favourable version of the auxiliary device the supporting structure is an aiming unit known in itself.

The most important advantage of the auxiliary device according to the invention is that, with the use of it, it becomes possible without the risk of radiation exposure to determine the positions of locking holes situated at the so-called "distal end" of long intramedullary nails that has become deformed in the course of being inserted with the desired precision, and to insert locking screws in a faster and more protective way. A further advantage deriving from this is that in an environment free from radiation exposure it is possible to perform longer operations without interruptions, without endangering the patient's or the operating surgeon's health. A further advantage resulting from this is that the time limit that operating surgeons can spend in the operating theatre can be extended, which may have a favourable effect on the course of medical attendance.

It is also an advantage of the auxiliary device that the supporting structure of the external positioning part-unit is basically the commonly used aiming device, which makes it easy to use and handle and also reduces the number of instruments used during the operations and the time needed for performing operations. A further advantage deriving from this is that in order to determine the exact position of locking holes there is no need for an expensive and complex device, which may also reduce the cost of operations.

In the following the invention is presented in detail in connection with a construction example on the basis of a drawing. On the drawing
Figure 1 shows a possible construction of the auxiliary device according to the invention in side view in partial cross-section, with an enlarged representation of the intramedullary nail,
Figure 2 is a reduced image of the cross-section of figure 1 in the direction II-II.

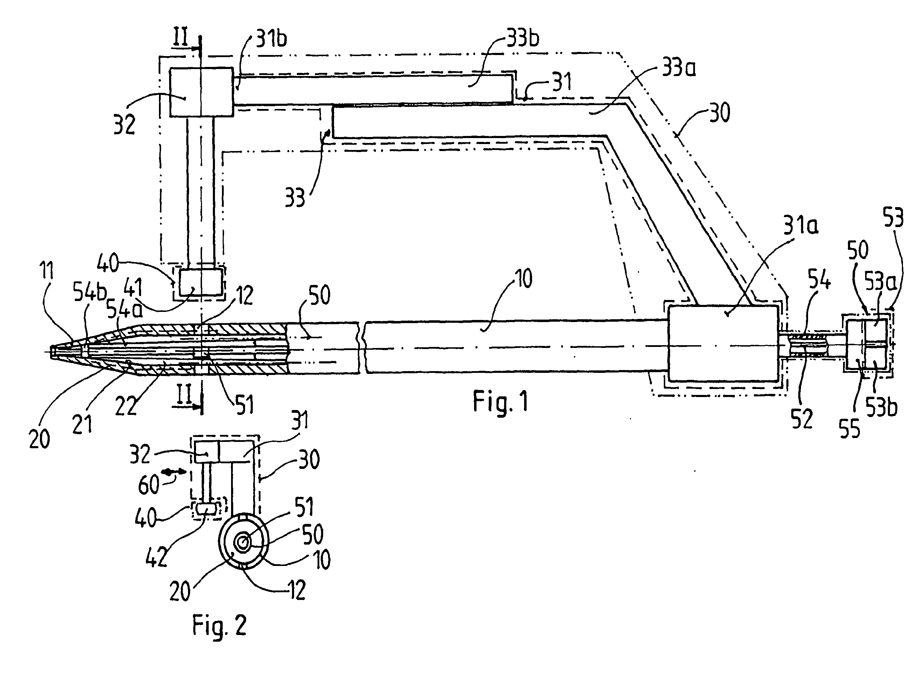

Figure 1 shows a version of the auxiliary device according to the invention containing an intramedullary nail 10 used for the restoration of a broken thighbone. The intramedullary nail 10, as a base body, comprises the internal positioning part-unit 20 on the one part, and on the other part it has connection to the external positioning part-unit 30. Here the internal positioning part-unit 20 is a guide sleeve 22 situated inside the intramedullary nail 10, which is hollow anyway, running along its whole length, and it also has an orientation piece 21 in the vicinity of the entrance point 11 of the intramedullary nail 10. Here the orientation piece 21 is constructed in the vicinity of the entrance point 11, as an envelope of cone the cross-section of which is decreasing towards the entrance point 11.

In the case of the present construction example in the guide sleeve 22 of the intramedullary nail 10 belonging to the internal positioning part-unit 20 there is a carrying body 54 belonging to the sensor unit 50. On the part of the covering surface 54a of the carrying body in the vicinity of the entrance point 11 of the intramedullary nail 10 there is a locking piece 54b, which in co-operation with the orientation piece 21 of the internal positioning part-unit 20 stops the carrying body 54 of the sensor unit 50 in a position where the relay unit 51 fitted in the carrying body 54 falls exactly in the dividing plane and/or opening of the locking hole 12 of the intramedullary nail 10.

The sensor unit 50 also contains a display unit 53 and an energy source 55 situated near the display unit 53 with the task of providing energy for the display unit 53. The display unit 53 may be a light emitting member 53a or a sound emitting member 53b or both.

Figure 1 shows that the display unit 53 and the energy source 55, similarly to the relay unit 51, are attached to the carrying body 54 of the sensor unit 50, and they are situated at the end of the carrying body 54 opposite the locking piece 54b. Data traffic between the relay unit 51 and the display unit 53 is ensured by an information transmission line 52. In this version the information transmission line is a cable also attached to the carrying body 54.

Figure 1 also shows that the fixing end 31a of the supporting structure 31 of the external positioning part-unit 30 is connected to the end of the intramedullary nail 10 opposite to the entrance point 11, while the free end 31b of the supporting structure 31 is connected to the motion member 32. The motion member 32 carries the source unit 40, which in this case is a natural magnet 41. Basically in the shown construction example of the auxiliary device the supporting structure 31 is an aiming unit 33 constructed from a proximal aiming device known in itself and a distal aiming device 33b also known in itself, co-operating with the proximal aiming device 33a.

As a result of this in the case of this solution at the fixing end 31a of the supporting structure 31 the intramedullary nail 10 is connected to the proximal aiming device 33a of the aiming unit 33 in the usual way seen in other uses. The supporting structure 31 and the intramedullary nail are attached to each other in a fixed position at a fixed angle, while the task of the motion member 32 situated at the free end 31b of the supporting structure is to enable the movement of the supporting structure 31 and the source unit in relation to each other along a certain plane.

In figure 2 it can also be seen that the supporting structure 31 of the external positioning part-unit 30 and the motion member 32 and the source unit 40 connected to it are connected to each other in a way that the source unit 40 can be moved in the motion direction 60 shown with an arrow beside the intramedullary nail 10 and accordingly beside the relay unit 51 of the sensor unit 50 situated in the internal positioning part-unit 20 of the intramedullary nail 10. It must be pointed out that here the source unit 40 contains the radiation source 42, which practically emits soft gamma radiation.

On the basis of the above it is obvious that the source unit 40 and the relay unit 51 of the sensor unit 50 must be chosen so that they are able to co-operate with each other, that is the relay unit 51 can evidently and reliably receive the signals emitted by the source unit 40, and only these signals. So in the case that the source unit 40 has a natural magnet 41, the relay unit 51 of the sensor unit 50 must sense the varying magnetic field, while in the case that the source unit is equipped with a radiation source 42 the relay unit 51 must sense the certain intensity of this radiation.

The pairing of sensor unit 50 - source unit 40 is also possible, where the sensor unit 50 is only for breaking or deforming the magnetic field created by the natural magnet 41 of the source unit 40, and this varying field is sensed by the source unit 40 and it can give a signal at the same time. This version is based on the principle of metal locators.

It must also be pointed out that not only the aiming unit 33 can be the supporting structure 31 of the external positioning part-unit 30, but using the known aiming units 33 as supporting structures 31 also significantly simplifies the operation of the auxiliary device, which is very important from the aspect of the operating surgeon.

In the course of the use of the auxiliary device according to the invention first the intramedullary nail must be placed in the medullary space of the broken bone to be restored. When this part of the operation has been completed, the carrying body 54 of the sensor unit 50 is inserted in the guide sleeve 22 of the internal positioning part-unit 20 situated in the intramedullary nail 10. In the guide sleeve 22 the carrying body 54 advancing towards the entrance point 11 of the intramedullary nail 10 slides into the guide sleeve 22 until the locking piece 54b protruding from the covering surface 54a of the carrying body 54 is knocked against the orientation piece 21 of the internal positioning part-unit 20. When the orientation piece 21 is touched by the locking piece 54b, the carrying body 54 cannot be pushed any further into the guide sleeve 22 of the internal positioning part-unit 20, which also means that the relay unit 51 of the carrying body 54 - as shown in figure 1 - is now situated right aligned with the locking hole 12 of the intramedullary nail 10.

After inserting the carrying body 54 of the sensor unit 50 the motion member 32 equipped with the source unit 40 is put on the free end 31b of the supporting structure 31 attached to the intramedullary nail 10, and with the help of the motion member 32 the source unit 40 is moved beside the intramedullary nail from the free end 31b of the supporting structure 31, from left to right looking at the arrangement according to figure 2. In the course of the movement of the source unit 40 first it approaches the intramedullary nail 10, and when it is close enough for the force of the magnetic field of the natural magnet 41 or the radiation of the radiation source 42 to reach the relay unit 51 situated in the intramedullary nail, it is switched on. With a command sent through the information transmission line 52 the relay unit 51 put into operation starts up the display unit 53, on which the light emitting member 53a starts flashing and the sound emitting member 53b starts whistling.

These signals continue until the source unit 40, after passing the intramedullary nail 10, gets so far from the free end 31b of the supporting structure 31 to the right of the intramedullary nail 10 that the signals emitted by the source unit 40 cannot be sensed by the relay unit 51 of the sensor unit 5 any more. At this point the display unit 53 is turned off and the light signals of the light emitting member 53a and the whistling sound of the sound emitting member 53b is discontinued.

The exact position of the intramedullary nail 10 can be easily determined knowing the points where the signals are turned on and off. Due to the symmetrical, homogenous radiation of the source unit 40 the position of the intramedullary nail 10 is in the middle of the distance between the points where the signals are turned on and off. If the distal aiming device 33b of the aiming unit 33 functioning as a supporting structure 31 so far is set to this distance, a hole suiting the locking hole 12 of the intramedullary nail 10 can be made with the desired precision.

Obviously there are other ways of precise setting too. In the case of a possible version the locking bore 12 of the intramedullary nail 10 can be found by sensing a marginal or a certain point of the force field, without halving the distance as described above.

The auxiliary device according to the invention can be favourably used in surgical interventions where the locking holes of intramedullary nails placed in tissues or bones must be found with an external drill.

**List of references**

| | |
|---|---|
| 10 intramedullary nail | 11 entrance point |
| | 12 locking hole |
| | |
| 20 internal positioning part-unit | 21 orientation piece |
| | 22 guide sleeve |
| | |
| 30 external positioning part-unit | 31 supporting structure |
| | 31a fixing end |
| | 31b free end |
| | 32 motion member |
| | 33 aiming unit |
| | 33a proximal aiming device |
| | 33b distal aiming device |
| | |
| 40 source unit | 41 natural magnet |
| | 42 radiation source |
| | |
| 50 sensor unit | 51 relay unit |
| | 52 information transmission line |
| | 53 display unit |
| | 53a light emitting member |
| | 53b sound emitting member |
| | 54 carrying body |
| | 54a covering surface |
| | 54b locking piece |
| | 55 energy source |
| | |
| 60 motion direction | |

## Claims

1. Auxiliary device for the determination of the positions of the holes (12) of intramedullary nails (10), which contains a source unit (40) suitable for emitting signals penetrating body tissues, a sensor unit (50) co-operating with the source unit (40) and an internal positioning part-unit (20) that can be connected to the intramedullary nail (10), either the source unit (40) or the sensor unit (50) is attached to the internal positioning part-unit (20), and the sensor unit (50) is provided with a relay unit (51) for receiving the signals of the source unit (40) and a display unit (53) connected to the relay unit (51) through an information transmission line (52), **characterised by** that it is provided with an external positioning part-unit (30) that can be connected to the intramedullary nail (10), the external positioning part-unit (30) has a supporting structure (31) with a fixing end (31a) and a free end (31b) and a motion member (32) that can be connected to the free end (31b) of the supporting structure (31), the fixing end (31a) of the supporting structure (31) is attached to the intramedullary nail (10) in a fixed position, while the other from the source unit (40) and the sensor unit (50) is attached to the motion member (32) of the external positioning part-unit (30), so the relay unit (51) of the sensor unit (50) and the source unit (40) are interlinked to each other that is suitable for the continuous control and the exact determination of the relative position between the sensor unit (50) and the source unit (40) by the aid of the external positioning part-unit (30) that is connected to the intramedullary nail (10).

2. Auxiliary device as in claim 1, **characterised by** that the internal positioning part-unit (20) is a guide sleeve (22) situated in the vicinity of the entrance point (11) of the intramedullary nail (10), equipped with an orientation piece (21), running along at least a part of the length of the intramedullary nail (10).

3. Auxiliary device as in claim 2, **characterised by** that the sensor unit (50) is supplemented with a carrying body (54) comprising the relay unit (51) and the display unit (53), and the carrying body (54) has a covering surface (54a) suiting the shape and size of the guide sleeve (22), which can be at least partly inserted in the guide sleeve (22).

4. Auxiliary device as in claim 3, **characterised by** that the covering surface (54a) of the carrying body (54) of the sensor unit (50) is equipped with a locking piece (54b) co-operating with the orientation piece (21) of the guide sleeve (22) of the internal positioning part-unit (20) situated in the intramedullary nail (10).

5. Auxiliary device as in any of claims 1-4, **characterised by** that the display unit (53) of the sensor unit (50) is provided with a light emitting member (53a) and/or a sound emitting member (53b).

6. Auxiliary device as in any of claims 1-5, **characterised by** that the sensor unit (50) is provided with an energy source (55).

7. Auxiliary device as in any of claims 1-6, **characterised by** that the source unit (40) has a natural magnet (41) creating a magnetic field.

8. Auxiliary device as in any of claims 1-6, **characterised by** that the source unit (40) has a radiation source (42) suitable for emitting radiation with a wavelength penetrating tissues.

9. Auxiliary device as in any of claims 1-8, **characterised by** that the supporting structure (31) is an aiming unit (33) known in itself.

## Patentansprüche

1. Hilfsvorrichtung zur Bestimmung der Positionen der Löcher (12) intra-medullarer Nägel (10), die eine Quelleneinheit (40) aufweist, die zur Abgabe von Signalen geeignet ist, die Körpergewebe durchdringen, eine Sensoreinheit (50), die mit der Quelleneinheit (40) zusammenarbeitet, sowie eine innere Positionierungsteileinheit (20), die mit dem intra-medullaren Nagel (10) verbunden werden kann, wobei entweder die Quelleneinheit (40) oder die Sensoreinheit (50) an der inneren Positionierungsteileinheit (20) angebracht ist, und wobei die Sensoreinheit (50) mit einer Relaiseinheit (51) für ein Empfangen der Signale der Quelleneinheit (40) und einer Anzeigeeinheit (53) versehen ist, die über eine Informationsübertragungsleitung (52) mit der Relaiseinheit (51) verbunden ist, **dadurch gekennzeichnet, dass** sie mit einer äußeren Positionierungsteileinheit (30) versehen ist, die mit dem intra-medullaren Nagel (10) verbunden werden kann, wobei die äußere Positionierungsteileinheit (30) eine Trägerstruktur (31) mit einem Befestigungsende (31 a) und einem freien Ende (31b) sowie ein Bewegungselement (32) aufweist, das mit dem freien Ende (31b) der Trägerstruktur (31) verbunden werden kann, wobei das Befestigungsende (31a) der Trägerstruktur (31) an dem intra-medullaren Nagel (10) in einer festen Position angebracht ist, während die andere der Quelleneinheit (40) und der Sensoreinheit (50) an dem Bewegungselement (32) der äußeren Positionierungsteileinheit (30) angebracht ist, so dass die Relaiseinheit (51) der Sensoreinheit (50) und die Quelleneinheit (40) miteinander verbunden sind, was für die kontinuierliche Steuerung und die exakte Bestimmung der relativen Position zwischen der Sensoreinheit (50) und der Quelleneinheit (40) mithilfe der äußeren Positionierungsteileinheit (30) geeignet ist, die mit dem intra-medullaren Nagel (10) verbunden ist.

2. Hilfsvorrichtung nach Anspruch 1, **dadurch gekennzeichnet, dass** die innere Positionierungsteileinheit (20) eine in der Nähe des Eingangspunkts (11) des intra-medullaren Nagels (10) angeordnete Führungshülse (22) ist, die mit einem Ausrichtungsteil (21) ausgestattet ist, und die entlang mindestens eines Teils der Länge des intra-medullaren Nagels (10) verläuft.

3. Hilfsvorrichtung nach Anspruch 2, **dadurch gekennzeichnet, dass** die Sensoreinheit (50) mit einem die Relaiseinheit (51) und die Anzeigeeinheit (53) aufweisenden Trägerkörper (54) ergänzt ist, wobei der Trägerkörper (54) eine zu der Form und Größe der Führungshülse (22) passende Abdeckfläche (54a) aufweist, die mindestens teilweise in die Führungshülse (22) eingeführt werden kann.

4. Hilfsvorrichtung nach Anspruch 3, **dadurch gekennzeichnet, dass** die Abdeckfläche (54a) des Trägerkörpers (54) der Sensoreinheit (50) mit einem Verriegelungsteil (54b) ausgestattet ist, das mit dem Ausrichtungsteil (21) der Führungshülse (22) der in dem intra-medullaren Nagel (10) angeordneten inneren Positionierungsteileinheit (20) zusammenarbeitet.

5. Hilfsvorrichtung nach einem der Ansprüche 1 bis 4, **dadurch gekennzeichnet, dass** die Anzeigeeinheit (53) der Sensoreinheit (50) mit einem lichtemittierenden Element (53a) und/oder einem schallemittierenden Element (53b) versehen ist.

6. Hilfsvorrichtung nach einem der Ansprüche 1 bis 5, **dadurch gekennzeichnet, dass** die Sensoreinheit (50) mit einer Energiequelle (55) versehen ist.

7. Hilfsvorrichtung nach einem der Ansprüche 1 bis 6, **dadurch gekennzeichnet, dass** die Quelleneinheit (40) einen natürlichen Magneten (41) aufweist, der ein magnetisches Feld erzeugt.

8. Hilfsvorrichtung nach einem der Ansprüche 1 bis 6, **dadurch gekennzeichnet, dass** die Quelleneinheit (40) eine Strahlungsquelle (42) aufweist, die dafür geeignet ist, Strahlung mit einer Wellenlänge zu emittieren, die Gewebe durchdringt.

9. Hilfsvorrichtung nach einem der Ansprüche 1 bis 8, **dadurch gekennzeichnet, dass** die Trägerstruktur (31) eine an sich bekannte Zieleinheit (33) ist.

## Revendications

1. Dispositif auxiliaire pour la détermination des positions des trous (12) de clous intramédullaires (10), qui contient une unité de source (40) appropriée pour émettre des signaux pénétrant dans les tissus corporels, une unité de capteur (50) coopérant avec l'unité de source (40) et une unité de partie de positionnement interne (20) qui peut être reliée au clou intramédullaire (10), soit l'unité de source (40), soit l'unité de capteur (50) étant fixée à l'unité de partie de positionnement interne (20), et l'unité de capteur (50) étant pourvue d'une unité de relais (51) pour recevoir les signaux de l'unité de source (40) et d'une unité d'afficheur (53) connectée à l'unité de relais (51) par l'intermédiaire d'une ligne de transmission d'informations (52), **caractérisé en ce qu'**il est pourvu d'une unité de partie de positionnement externe (30) qui peut être reliée au clou intramédullaire (10), l'unité de partie de positionnement externe (30) comporte une structure de support (31) avec une extrémité de fixation (31a) et une extrémité libre (31b) et un élément de mouvement (32) qui peut être relié à l'extrémité libre (31b) de la structure de support (31), l'extrémité de fixation (31a) de la structure de support (31) est fixée au clou intramédullaire (10) dans une position fixée, tandis que l'autre de l'unité de source (40) et de l'unité de capteur (50) est fixée à l'élément de mouvement (32) de l'unité de partie de positionnement externe (30), ainsi l'unité de relais (51) de l'unité de capteur (50) et l'unité de source (40) sont reliées l'une à l'autre, ce qui est approprié pour la commande continue et la détermination exacte de la position relative entre l'unité de capteur (50) et l'unité de source (40) à l'aide de l'unité de partie de positionnement externe (30) qui est reliée au clou intramédullaire (10).

2. Dispositif auxiliaire selon la revendication 1, **caractérisé en ce que** l'unité de partie de positionnement interne (20) est un manchon de guidage (22) situé dans le voisinage du point d'entrée (11) du clou intramédullaire (10), équipé d'une pièce d'orientation (21), s'étendant le long d'au moins une partie de la longueur du clou intramédullaire (10).

3. Dispositif auxiliaire selon la revendication 2, **caractérisé en ce que** l'unité de capteur (50) est complétée par un corps de support (54) comprenant l'unité de relais (51) et l'unité d'afficheur (53), et le corps de support (54) comporte une surface de recouvrement (54a) adaptée à la forme et à la taille du manchon de guidage (22), qui peut être au moins partiellement insérée dans le manchon de guidage (22).

4. Dispositif auxiliaire selon la revendication 3, **caractérisé en ce que** la surface de recouvrement (54a) du corps de support (54) de l'unité de capteur (50) est pourvue d'une pièce de verrouillage (54b) coopérant avec la pièce d'orientation (21) du manchon de guidage (22) de l'unité de partie de positionnement interne (20) située dans le clou intramédullaire (10).

5. Dispositif auxiliaire selon l'une quelconque des revendications 1 à 4, **caractérisé en ce que** l'unité d'afficheur (53) de l'unité de capteur (50) est pourvue d'un élément émettant de la lumière (53a) et/ou d'un élément émettant un son (53b).

6. Dispositif auxiliaire selon l'une quelconque des revendications 1 à 5, **caractérisé en ce que** l'unité de capteur (50) est pourvue d'une source d'énergie (55).

7. Dispositif auxiliaire selon l'une quelconque des revendications 1 à 6, **caractérisé en ce que** l'unité de source (40) comporte un aimant naturel (41) créant un champ magnétique.

8. Dispositif auxiliaire selon l'une quelconque des revendications 1 à 6, **caractérisé en ce que** l'unité de source (40) comporte une source de rayonnement (42) appropriée pour émettre un rayonnement avec une longueur d'onde pénétrant dans les tissus.

9. Dispositif auxiliaire selon l'une quelconque des revendications 1 à 8, **caractérisé en ce que** la structure de support (31) est une unité de visée (33) connue en elle-même.
